# EUROPEAN PATENT SPECIFICATION

(11) **EP 1 214 108 B1**
(45) Date of publication and mention of the grant of the patent: **10.01.2007**
(21) Application number: 00957860.0
(22) Date of filing: 23.08.2000
(51) Int. Cl.: A61L 31/14, A61L 31/16

(54) **A POROUS PROSTHESIS AND A METHOD OF DEPOSITING SUBSTANCES INTO THE PORES**
PORÖSE PROTHESE UND VERFAHREN ZUR ABSCHEIDUNG VON SUBSTANZEN IN DEN POREN
PROTHESE POREUSE ET PROCEDE DE DEPOT DE SUBSTANCES DANS LES PORES

(30) Priority: 03.09.1999 US 390855; 03.09.1999 US 390069
(43) Date of publication of application: 19.06.2002
(73) Proprietor: ADVANCED CARDIOVASCULAR SYSTEMS, INC., Santa Clara, CA 95054-2807 (US)
(72) Inventor: HOSSAINY, Syed, F., A., Fremont, CA 94555 (US); CHEN, Li, San Jose, CA 95138 (US)
(74) Representative: Price, Nigel John King
(86) International application number: PCT/US2000/023535
(87) International publication number: WO 2001/017577

(56) References cited:
- EP-A- 0 850 651
- EP-A- 0 875 218
- WO-A-99/16386
- PATENT ABSTRACTS OF JAPAN vol. 2000, no. 02, 29 February 2000 (2000-02-29) & JP 11 299901 A (JOHNSON &JOHNSON MEDICAL KK), 2 November 1999 (1999-11-02)

## Description

### BACKGROUND OF THE INVENTION

### Field of the Invention

This invention relates to implantable devices, such as an expandable, intraluminal prosthesis commonly known as a stent. More particularly, this invention relates to a prosthesis having pores formed in its cylindrical body. Moreover, the present invention relates to a method of depositing substances, such as therapeutic substances, in the pores.

### Description of the Background

Percutaneous transluminal coronary angioplasty (PTCA) is a procedure for treating heart disease. A catheter assembly having a balloon portion is introduced into the cardiovascular system of a patient via the brachial or femoral artery. The catheter assembly is advanced through the coronary vasculature until the balloon portion is positioned across the occlusive lesion. Once in position across the lesion, the balloon is inflated to a predetermined size to radially compress the atherosclerotic plaque of the lesion against the inner wall of the artery to dilate the lumen. The balloon is then deflated to a smaller profile to allow the catheter to be withdrawn from the patient's vasculature.

A problem associated with the procedure includes formation of intimal flaps or torn arterial linings which can collapse and occlude the conduit after the balloon is deflated. Moreover, thrombosis and restenosis of the artery may develop over several months after the procedure, which may require another angioplasty procedure or a surgical by-pass operation. To reduce the partial or total occlusion of the artery by the collapse of arterial lining and to reduce the chance of the development of thrombosis and restenosis, an intraluminal prosthesis, an example of which includes an expandable stent, is implanted in the lumen to maintain the vascular patency. Stents are scaffoldings, usually cylindrical or tubular in shape, functioning to physically hold open, and if desired, to expand the wall of the passageway. Typically stents are capable of being compressed for insertion through small cavities via small catheters, and then expanded to a larger diameter once at the desired location. Examples in patent literature disclosing stents which have been successfully applied in PTCA procedures include stents illustrated in U.S. Patent No. 4,733,665 issued to Palmaz, U.S. Patent No. 4,800,882 issued to Gianturco, and U.S. Patent No. 4,886,062 issued to Wiktor.

In treating the damaged vasculature tissue and to further fight against thrombosis and restenosis, there is a need for administrating therapeutic substances to the treatment site. For example, anticoagulants, antiplatelets and cytostatic agents are commonly used to prevent thrombosis of the coronary lumen, to inhibit development of restenosis, and to reduce post-angioplasty proliferation of the vascular tissue, respectively. To provide an efficacious concentration to the treated site, systemic administration of such medication often produces adverse or toxic side effects for the patient. Local delivery is a highly suitable method of treatment in that smaller levels of medication, as compared to systemic dosages, are concentrated at a specific site. Local delivery produces fewer side effects and achieves more effective results.

One commonly applied technique for the local delivery of the drugs is through the use of medicated stents. One proposed method of medicating stents is to seed the stent with endothelial cells (Dichek, D.A. et al. Seeding of Intravascular Stents With Genetically Engineered Endothelial Cells; Circulation 1989; 80: 1347-1353). Briefly, endothelial cells can be seeded onto stainless steel stents and grown until the stents are covered. The cells are therefore able to be delivered to the vascular wall to provide therapeutic proteins. Another proposed method of providing therapeutic substances to the vascular wall includes simple heparin-coated metallic stent, whereby a heparin coating is ionically or covalently bonded to the stent. Disadvantages associated with the aforementioned methods include significant loss of the therapeutic substance from the body of the stent during delivery and expansion of the stent and an absolute lack of control of the release rate of the therapeutic substance from the stent. Another proposed method involves the use of a polymeric carrier coated onto the body of the stent, as disclosed in U.S. Patent No. 5,464,650 issued to Berg et al., U.S. Patent No. 5,605,696 issued to Eury et al., U.S. Patent No. 5,865,814 issued to Tuch, and U.S. Patent No. 5,700,286 issued to Tartaglia et al. Obstacles often encountered with the use of a polymeric coating include difficulties in coating a complicated geometrical structure, poor adhesion of the polymeric coating to the surface of a stent, and biocompatibility of the polymer. Accordingly, it is desirable to be able to secure the therapeutic substance directly onto the body of the stent. Not withstanding the benefits gained by securing a therapeutic substance to the body of the stent, it is also desirable to be able to secure other substances to the body of the stent, such as radiopaque materials, used to assist a physician to guide and deploy the stent at the proper site of treatment.

EP-A-0 875 218 discloses a medicated prosthesis, such as a stent, for deployment in a human vessel. A metallic stent has a plurality of pores in the metal which are loaded with medication. The medication is loaded into the pores by applying to the stent a liquid comprising the medication dissolved in a solvent or suspended in a liquid mixture.

### SUMMARY OF THE INVENTION

According to a first aspect of the present invention there is provided a method of loading a substance into pores of an implantable prosthesis, the method comprising:
providing a prosthesis having a surface and pores formed in said surface;
applying to said prosthesis a first fluid having an added substance, wherein during said act of applying said first fluid penetrates into said pores;
removing said first fluid from said prosthesis substantially to elimination;
applying to said prosthesis a second fluid to remove said substance from said1 surface of said prosthesis, said second fluid having a contact angle greater than about 90° so as to prevent said second fluid from significantly penetrating into said pores to remove said substance out from said pores during the application of said second fluid; and
removing said second fluid from said prosthesis substantially to elimination,
wherein said substance is deposited into said pores.

According to a second aspect of the present invention there is provided an implantable prosthesis, comprising:
a body structure having a generally cylindrical shape, an outer surface capable of contacting an inner lumen surface of a passageway, and a hollow bore which extends longitudinally through said body structure to define an inner surface, said body structure having a thickness;
said body structure having a plurality of depots formed on at least a region of said outer surface, said depots having a depth less than said thickness of said body structure, and said depots having a preselected and controlled location and a preselected and controlled depth.

In the hereinafter described and illustrated preferred embodiments of implantable prosthesis, the resultant prosthesis is a stent defined by a cylindrical shaped body having a thickness. Depots or pores are formed on the body at preselected locations. The depots have a preselected depth and shape. The depth of the depots can be equal to 10% to 90% of the thickness. In one embodiment the depots can have a cylindrical shape. In another embodiment the shape can be generally conical. Substances such as therapeutic substances, polymeric material, polymeric material containing therapeutic substances, radioactive isotopes, and radiopaque material can be deposited into the depots.

In a preferred method in accordance with the present invention a first fluid having a substance added therein is applied to a porous prosthesis. During the application, the first fluid containing the substance is capable of penetrating into the pores. The first fluid is removed, for example by evaporation, and a second fluid is applied to the prosthesis. During the application of the second fluid, the second fluid is not capable of significantly penetrating into the pores. The second fluid can have a contact angle greater than about 90°. Contact angle is defied as the angle at the tangent of the fluid phase that has taken an equilibrium shape on a solid surface. In one embodiment of the present invention, the second fluid rinses the substance from the surface of the body of the prosthesis. In another embodiment, a therapeutic substance and/or a polymer can be added to the second fluid to form a coating of a therapeutic substance and/or polymer onto the surface of the body of the prosthesis.

In accordance to another embodiment, prior to the application of the second fluid, the prosthesis can be immersed in a third fluid and agitated via mechanical perturbation techniques. Accordingly, any of the substance gathered on the surface of the body after the application of the first fluid is removed. The third fluid should not be capable of dissolving the substance. The third fluid can have a contact angle above 90°.

### BRIEF DESCRIPTION OF THE DRAWINGS

The features of the described embodiments are specifically set forth in the appended claims. However, embodiments relating to both structure and method of operation are best understood by referring to the following description and accompanying drawings, in which similar parts are identified by like reference numerals.
Figure 1 is a side view of a conventional intraluminal prosthesis, the body of the prosthesis being defined by cylindrical elements engaged to one another by connecting elements;
Figure 2 is an enlarged view of section A of Figure 1, illustrating a portion of the cylindrical elements and connecting elements;
Figure 3A is a cross sectional view of the cylindrical element, taken in the direction of the arrows and along the plane of line 3-3 of Figure 2, illustrating a depot formed in the body of the prosthesis in accordance to one embodiment of the present invention;
Figure 3B is a cross sectional view of the cylindrical element, taken in the direction of the arrow and along the plane of line 3-3 of Figure 2, illustrating a depot formed in the body of the prosthesis in accordance to another embodiment of the present invention;
Figure 4A illustrates a fluid on a solid surface having a contact angle Φ₁; and
Figure 4B illustrates a fluid on a solid surface having a contact angle Φ₂.

### DETAILED DESCRIPTION OF THE PREFERRED EMBODIMENTS

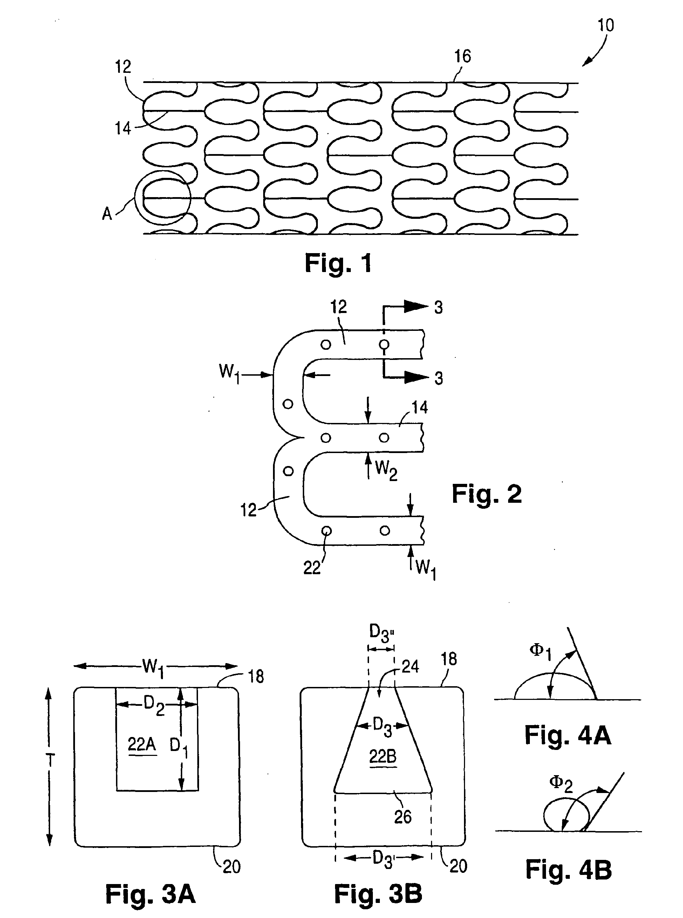

Figure 1 illustrates an implantable prosthesis 10, one example of which includes a stent. Stents are scaffoldings, usually cylindrical or tubular in shape, that are inserted into a anatomical passageway and operate to physically hold open and, if desired, to expand the wall of a passageway. Stents are capable of being compressed for insertion through small cavities via balloon-catheters, positioned in a desired location, then expanded to a larger diameter.

In one example, illustrated in Figures 1 and 2, stent 10 includes a plurality of rigid but resiliently flexible thread elements 12 that are arranged in a sinusoid-like configuration that is connected to form a continuous ring or cylinder. The plurality of cylindrical thread elements 12 are radially expandable, disposed coaxially, and interconnected by connecting elements 14 that are disposed between adjacent cylindrical thread elements 12, leaving gaps or lateral openings between adjacent cylindrical thread elements 12. Although the thread elements 12 are illustratively shown in the form of cylinders or rings connected axially-displaced in-parallel, other configurations, such as helices, coils, or braids, and other connections may be utilized. Thread elements 12 and connecting elements 14 define a tubular stent body 16 having a lumen contacting surface 18 and an inner surface 20 as shown in Figures 3A and 3B.

Thread elements 12 have any suitable width W₁, typically in a range of widths W₁ from 5,08·10⁻⁸m (0.002 inches) to 4,52·10⁻⁴m (0.006 inches). A common width W₁ is about 7,62·10⁻⁵m (0.003 inches). Connecting elements 14 have any suitable width W₂, typically in a ranges of widths W₂ from 5,08·10⁻⁸m (0.002 inches) to 2,03·10⁻⁴m (0.008 inches). A common width W₂ is about 1,27·10⁻⁴m (0.005 inches). Additionally, thread elements 12 have any suitable thickness T, typically a thickness T in a range from 5,08·10⁻⁸m (0.002 inches) to 2,03·10⁻⁴m (0.008 inches). A common thickness T is about 1,27·10⁻⁴m (0.005 inches). Connecting elements 14 have any suitable thickness, typically in a range from 5,08·10⁻⁸m (0.002 inches) to 2,03·10⁻⁴m (0.008 inches). A common connecting element 14 thickness is about 1,27·10⁻⁴m (0.005 inches). A specific choice of width and thickness depends on the anatomy and size of the target lumen. In other words, the size of the stent can vary according to intended procedure, anatomy, and usage.

In one embodiment, thread elements 12 and connecting elements 14 are typically fabricated from a metallic material or an alloy such as stainless steel (e.g., 316L), "MP35N," "MP20N," tantalum, nickel-titanium alloy (commercially available as Nitinol™), platinum-iridium alloy, gold, magnesium, or combinations of alloys. "MP35N" and "MP20N" are trade names for alloys of cobalt, nickel, chromium and molybdenum available from standard Press Steel Co., Jenkintown, PA. "MP35N" has a nominal composition of 35% cobalt, 35% nickel, 20% chromium, and 10% molybdenum. "MP20N" has a nominal composition of 50% cobalt, 20% nickel, 20% chromium, and 10% molybdenum. The aforementioned list is merely a useful list of materials and that other materials are proven to function effectively.

A single depot or pore 22 or plurality of depots or pores 22 are formed as a laser trench or laser trenches on body 16 of stent 10 by exposing surface 18 to an energy discharge from a laser, such as an excimer laser. Alternative methods of forming depots 22 include physical or chemical etching techniques. Techniques of laser fabrication or etching to form depots 22 are well-known to one of ordinary skill in the art. Depots 22 can be formed in virtually any stent structure and not merely the above-described structure. The depots 22 are used for carrying a variety of substances including but not limited to therapeutic substances, polymers impregnated with therapeutic substances, radioactive isotopes, and radiopaque materials. The location of a depot 22 or depots 22 vary according to intended usage and application. Depots 22 are formed by a manufacturer at any preselected location and have any preselected depth, size, and geometrical configuration. In one example, depots 22 are evenly distributed through body 16 and have an equal volume so that the tissue in contact with stent 10 receives an equal distribution of a therapeutic substance. Depth D₁ of a depot 22 typically is varied in proportion to the thickness T of body 16 as well as the clinical purpose and usage.

For a stent 10 that carries a therapeutic substance or a polymeric carrier impregnated with a therapeutic substance, a suitable depot or pore depth D₁ has a range from 10% to 90 % of thickness T. Typically a depth not greater than about 50% of thickness T is most suitable. The specific depth D₁ of depots 22 depends on the amount of therapeutic substance that is to be deposited in depots 22. In an example of a stent 10 that carries a radioactive isotope, depth D₁ is typically 10% to 80% of thickness T. A more specific suitable depth is not greater than about 30% of thickness T is suitable.

For a stent 10 that carries a radiopaque material, a suitable depot or pore depth D₁ has a range from 10% to 90% of thickness T. Typically a depth not greater than about 65% is most suitable. A depth D₁ greater than about 65% of the thickness may compromise the structural integrity and mechanical functionality of stent 10. However the upper limit of depth D₁ varies depending on the material characteristics such as the hardness of the body 16.

Depots 22 may be formed in a variety of selected geometrical shapes. Referring to Figure 3A, a depot 22A is a generally cylindrical shape. A diameter D₂ of cylindrical depot 22A typically has a range from 10% to 90% of width W₁ or W₂, although the diameter D₂ is usually not greater than about 80% of width W₁ or W₂. The specific diameter D₂ depends on the application and purpose of the depots 22. The upper limit of diameter D₂ varies depending on the material characteristics such as the hardness of the body 16.

An alternative example of a depot 22B, illustrated in Figure 3B, is generally conical in shape. Conical shaped depot 22B has an open end 24 and a closed end 26. The open end 24 is the end that contacts a surface of a tissue. A diameter D₃ of conical shaped depot 22B is shown to decrease from closed end 26 to open end 24. The largest diameter D₃' at the closed end 26 of conical shaped depot 22B has a range from 10% to 80% of width W₁ or W₂. Generally, the largest diameter D₃' is not greater than about 70% of width W₁ or W₂. The smallest diameter D₃" at the open end 24 of conical shaped depot 22B has a range from 5% to 70% of width W₁ or W₂. Generally, the smallest diameter D₃" is not greater than about 60% of width W₁ or W₂. The reduced size of opening 24 of conical shaped depot 22B, as compared to that of the cylindrical shaped depot 22A, reduces the rate at which a therapeutic substance is released once the stent is implanted at the desired location of treatment. The depots 22 can have a variety of other geometrical shapes, such as elongated trenches (not illustrated).

The depth D₁ and diameters D₂ and D₃ of the individual depots 22 formed on body 16 of stent 10 can vary relative to one another. In one example, the manufacturer selectively controls the volume of depots on different positions of the body 16, either selectively varying the volume or making the volume consistent throughout the body 16. For some applications, consistent depot 22 volume is important for delivery of a therapeutic substance to insure that the substance is evenly distributed throughout stent 10 and results in consistent application of the therapeutic substance to the tissues in contact with surface 18 of stent 10.

A factor for determining the size, geometry, and concentration of depots 22 is the overall porosity of stent 10. Porosity is the total volume of pores in the body 16 of stent 10 divided by the total volume of structural material of the stent 10. Porosity determines the capacity of substance that can be loaded into a stent 10 of predetermined dimensions. High porosity can adversely affect the structural integrity, strength, and elasticity of the stent 10. Consequently, stent design includes consideration of a tradeoff between strength, on one hand, and stent profile and stent load capacity on the other hand.

Substances are deposited into the depots or pores 22 using several illustrative methods. The methods are applicable to the illustrative stents 10 described hereinbefore and also to any type of porous prosthesis. In some examples, the deposited substance is a therapeutic substance or agent such as antineoplastics, antiinflammatory substances, antiplatelets, anticoagulants, fribrinolytics, thrombin inhibitors, antimitotics, and antiproliferatives. Examples of antineoplastics include paclitaxel and docetaxel. Examples of antiplatelets, anticoagulants, fribrinolytics, and thrombin inhibitors include sodium heparin, low molecular weight heparin, hirudin, argatroban, forskolin, vapiprost, prostacyclin and prostacyclin analogues, dextran, D-phe-pro-arg-chloromethylketone (synthetic antithrombin), dipyridamole, glycoprotein IIb/IIIa platelet membrane receptor antibody, recombinant hirudin, thrombin inhibitor (available from Biogen), and 7E-3B® (an antiplatelet drug from Centocore). Examples of suitable antimitotic agents include methotrexate, azathioprine, vincristine, vinblastine, flurouracil, adriamycin, and mutamycin. Examples of suitable cytostatic or antiproliferative agents include angiopeptin (a somatostatin analogue from Ibsen), angiotensin converting enzyme inhibitors such as Captopril® (available from Squibb), Cilazapril® (available from Hofman-LaRoche), or Lisinopril® (available from Merck); calcium channel blockers (such as Nifedipine), colchicine, fibroblast growth factor (FGF) antagonists, fish oil (omega 3-fatty acid), histamine antagonist, Lovastatin® (an inhibitor ofHMG-CoA reductase, a cholesterol lowering drug from Merck), monoclonal antibodies (such as PDGF receptors), nitroprusside, phosphodiesterase inhibitors, prostaglandin inhibitor (available form Glazo), Seramin (a PDGF antagonist), serotonin blockers, steroids, thioprotease inhibitors, triazolopyrimidine (a PDGF antagonist), and nitric oxide. Other therapeutic substances or agents which may be appropriate include alpha-interferon, genetically engineered epithelial cells, and dexamethasone. While the listed therapeutic substances or agents are well known for preventative and therapeutic utility, the substances are listed by way of example and are not meant to be limiting. Other therapeutic substances which are currently available or that may be developed in the future are equally applicable. The treatment of patients using the above mentioned medicines is well-known to those of ordinary skill in the art.

In other embodiments, the therapeutic substance is a radioactive isotope for stent usage in radiotherapeutic procedures. Examples of radioactive isotopes include, but are not limited to, phosphoric acid (H₃P³²O₄), palladium (Pd ¹⁰³), cesium (Cs ¹³¹), and iodine (I ¹²⁵).

A therapeutic substance is added to a first fluid or solvent. The therapeutic substance is dispersed throughout the first solvent so that it is in a true solution, saturated or supersaturated with the solvent or suspended in fine particles in the first solvent. If the therapeutic substance is suspended in particles in the first solvent, the pore size and the diameter of the opening of the pores are to be sufficiently large in comparison to the size of the particles to facilitate loading and unloading of the stent. In one example, suitable pores have a pore size that is more than ten times the particle size of a suspended therapeutic substance and an opening diameter that is more than five times the diameter of the particle size.

The first solvent can be virtually any solvent that is compatible with the therapeutic substance. A suitable first solvent typically has a high capillary permeation. Capillary permeation or wetting is the movement of fluid on a solid substrate driven by interfacial energetics. Capillary permeation is quantitated by a contact angle, defined as the angle at the tangent of the first solvent droplet in fluid phase that has taken an equilibrium shape on a solid surface. A low contact angle means a higher wetting liquid. A suitably high capillary permeation corresponds to a contact angle less than about 90°.

For example, Figure 4A illustrates a fluid having a high capillary permeation that corresponds to a solvent contact angle Φ₁ less than about 90°. Figure 4B illustrates a fluid having a low capillary permeation that corresponds to a fluid contact angle Φ₂ greater than about 90°. The first solvent can have a viscosity not greater than about 0.01 Pa.s (ten centipoise). A high capillary permeation and a viscosity not greater than about ten centipoise allows the first solvent to penetrate into the pores of the prosthesis more quickly, eliminating a requirement to apply the first solvent to the prosthesis for a prolonged period of time. The first solvent can be volatile, facilitating evaporation of the first solvent. Useful examples of some first solvent include, but are not limited to, acetone, ethanol, methanol, isopropanol, tetrahydrofuran, and ethyl acetate. The first solvent is applied to a porous prosthesis, for example by immersing or spraying the solvent in procedures that are well-known to one having ordinary skill in the art.

The first solvent is applied for a predetermined period of time, the specific time depending on the capillary permeation and viscosity of the first solvent, the volume of the pores, and the amount of substance to be deposited. Therapeutic parameters such as the concentration of the therapeutic substance in the solvent and dosages depend on the duration of local release, the cumulative amount of release, and desired rate of release. Correlations and interrelations between the therapeutic parameters are well-known to one having ordinary skill in the art and are simply calculated.

After applying the first solvent for a selected duration, the first solvent is removed from the prosthesis. In one example, the first solvent is removed by evaporation in ambient pressure, room temperature, and anhydrous atmosphere and/or by exposure to mild heat (e.g., 60° C) under a vacuum condition. The prosthesis typically has a clustered or gross formation of a therapeutic substance gathered on the body surface. The cluster is generally removed by immersing the prosthesis in a second fluid and agitating the prosthesis via mechanical perturbation techniques, such as vortexing or vigorous shaking. The second fluid is a non-solvent so that the therapeutic substance does not significantly dissolve in the second fluid. The non-solvent can have a low capillary permeation or a contact angle greater than about 90° and a viscosity not less than about 5.10⁻⁴ Pa.s (0.5 centipoise) so that the second fluid is not capable of significantly penetrating into the pores during the process of agitation. Examples of a second fluid include but are not limited to saturated hydrocarbons or alkanes, such as hexane, heptane, and octane.

The prosthesis is rinsed in a third fluid. The third fluid is typically a solvent to facilitate dissolution of the therapeutic substance. The third fluid generally has a low capillary permeation, corresponding to a contact angle greater than about 90°. The third fluid has a viscosity of not less than about 0,001 Pa.s (1.0 centipoise) and is therefore incapable of significantly penetrating into the pores during the rinsing stage. The rinsing is conducted rapidly for example in a range from 1 second to about 15 seconds, the exact duration depending on the solubility of the therapeutic substance in the solvent. Extended duration of exposure of the third solvent to the prosthesis may lead to the penetration of the third solvent into the pores.

The rinsing step is repeated, if desired, until all traces of therapeutic substance are removed from the surface of the stent. The third fluid removes excess traces of therapeutic substance from the surface of the prosthesis body. Useful examples of third fluids include but are not limited to dimethylsulfoxide (DMSO), water, DMSO in an aqueous solution, glyme, and glycerol. The third fluid is removed from the prosthesis body using a technique such as evaporation in ambient pressure, room temperature and anhydrous atmosphere and/or by exposure to mild heat (e.g., 60° C) under vacuum condition. The first, second, third fluids are selected to not adversely affect the characteristics and composition of the therapeutic substance.

In one embodiment, the third fluid can be highly volatile, for example having a boiling point of not greater than about 60° C at 1 atm. Accordingly, the third fluid is capable of rapidly evaporating. Rapid evaporation of the third fluid causes the third fluid to be removed from the prosthesis prior to any significant penetration of the third fluid in the pores. A useful example of a highly volatile third fluid includes, but is not limited to, Freon (e.g., Xerosolv™).

Once loaded, the therapeutic substance remains in the pores until prosthesis deployment and expansion. The expanded prosthesis engages the wall of the anatomical passageway and the therapeutic substance disseminates from the porous cavities and is absorbed into the tissue of the walls of the passageway that are in contact with the prosthesis.

In some embodiments, a surface of the stent is coated with a therapeutic substance in addition to having a therapeutic substance deposited in the pores. A coating of therapeutic substance on the surface of the prosthesis is formed by adding the therapeutic substance to the third fluid rinse. The therapeutic substance is dispersed through the third fluid to form a true solution with the third solvent, rather than a dispersion of fine particles. The therapeutic substance is a substance that is capable of absorbing or attaching to the prosthesis surface. For example, highly suitable therapeutic substances for a stainless steel prosthesis include taxol and dexamethasone. Suitable substances for a Nitinol^{™} prosthesis include aspirin and heparin. The therapeutic substance added to the third fluid is a different substance to the therapeutic substance deposited in the pores. Rinsing with the third fluid is optionally prolonged or repeated to increase the thickness of the prosthesis therapeutic substance coating.

In another example, a polymeric coating is formed on the surface of the prosthesis, covering the pores containing deposited therapeutic substance. The polymeric coating forms a membrane that reduces the rate of release of a therapeutic substance from the pores. A polymeric material is added to the third fluid rinse to form a coating made from the polymeric material on the prosthesis surface.

The polymeric material, by example and not limitation, forms 1% to 3% by weight of the total weight of the solution. The polymeric material is most suitably bio-compatible, including polymers that are non-toxic, non-inflammatory, chemically inert, and substantially non-immunogenetic in the applied amounts. The polymer is typically either be bioabsorbable or biostable. A bioabsorbable polymer bio-degrades or breaks down in the body and is not present sufficiently long after implantation to cause an adverse local response. Bioabsorbable polymers are gradually absorbed or eliminated by the body by hydrolysis, metabolic process, bulk, or surface erosion. Examples of bioabsorbable, biodegradable materials include but are not limited to polycaprolactone (PCL), poly-D, L-lactic acid (DL-PLA), poly-L-lactic acid (L-PLA), poly(lactide-co-glycolide), poly(hydroxybutyrate), poly(hydroxybutyrate-co-valerate), polydioxanone, polyorthoester, polyanhydride, poly(glycolic acid), poly(glycolic acid-cotrimethylene carbonate), polyphosphoester, polyphosphoester urethane, poly (amino acids), cyanoacrylates, poly(trimethylene carbonate), poly(iminocarbonate), copoly(ether-esters), polyalkylene oxalates, polyphosphazenes, polyiminocarbonates, and aliphatic polycarbonates. Biomolecules such as heparin, fibrin, fibrinogen, cellulose, starch, and collagen are typically also suitable. Examples of biostable polymers include Parylene®, Parylast®, polyurethane (for example, segmented polyurethanes such as Biospan®), polyethylene, polyethlyene teraphthalate, ethylene vinyl acetate, silicone and polyethylene oxide.

After the evaporation of the third solvent, a polymeric layer remains on the surface of the prosthesis and over the pores. The polymeric coating is alternatively formed by other conventional methods such as plasma polymerization, the practice of which is well known to one of ordinary skill in the art.

In another example, the aforementioned polymeric coating is impregnated with a therapeutic substance that is added to the third fluid-polymer mixture. Concentration of the therapeutic substance depends on release parameters including the duration of local release, the cumulative amount of release, and the desired rate of release. The correlations and interrelations between the release parameters are well-known to those of ordinary skill in the art and easily calculated. The polymeric material, by example and not limitation, makes up from 1% to 3% by weight of the total weight of the solution. The therapeutic substance, by example and not limitation, typically makes up 0.3% to 1% of the total weight of the solution. Once the third solvent is evaporated, a polymeric coating impregnated with a therapeutic substance remains on the surface, covering the therapeutic substance-filled pores.

In another example, a polymeric material, such as any of the polymeric materials listed herein, is impregnated with a therapeutic substance and deposited into the pores. The polymeric material reduces the rate of release of the therapeutic substance from the pores. The method of application includes adding a therapeutic substance and a polymer to a first fluid or solvent. The therapeutic substance is dispersed throughout the first solvent to dissolve into a true solution, or is saturated or supersaturated with the solvent or suspended in fine particles in the first solvent. The polymeric material is also dispersed throughout the first solvent to form a true solution, or is suspended in fine particles in the first solvent. Saturation or supersaturation of the polymer is less suitable since the viscosity of the first solvent is raised beyond a desired limit.

If the therapeutic substance is suspended in the first solvent, the pore size and the diameter of the opening of the pores are to be sufficiently large in comparison to the size of the particles to facilitate loading and unloading of the stent. In one example, suitable pores have a pore size that is more than ten times the particle size of a suspended therapeutic substance and an opening diameter that is more than five times the diameter of the particle size.

The first solvent is selected from among solvents that are compatible with the polymer and therapeutic substance. A first solvent having a high capillary permeation or a contact angle not higher than about 90° improves performance. The first solvent can have a viscosity not higher than about ten centipoise. The first solvent can be volatile to facilitate evaporation. Useful examples of a first solvent include but are not limited to acetone, ethanol, methanol, isopropanol, tetrahydrofuran, and ethyl acetate. The first solvent is applied to a porous prosthesis, by for example, immersing or spraying. The first solvent is applied for a predetermined time period, the specific time depending on the capillary permeation and viscosity of the first solvent, volume of the pores, and the amount of substance to be deposited. Therapeutic parameters such as the concentration of the therapeutic substance and the specific polymer depend on the duration of local release, the cumulative amount of release, and the rate of release that is desired. Correlations and interrelations between the therapeutic parameters are well-known to those having ordinary skill in the art and are easily determined.

After a selected time duration, the first solvent is removed from the prosthesis by a technique such as evaporation in ambient pressure, room temperature, and anhydrous atmosphere and/or by exposure to mild heat (e.g., 60° C) under vacuum condition. The prosthesis typically has a cluster of polymeric material and therapeutic substance formed on the body surface. The cluster is removed by immersing the prosthesis in a second fluid and agitating the prosthesis via mechanical perturbation techniques, such as vortexing or vigorous shaking. The second fluid is a non-solvent so that the therapeutic substance and the polymer are not capable of dissolution in the second fluid. The non-solvent generally has a low capillary permeation, corresponding to a contact angle greater than about 90° and a viscosity not less than 5.10⁻⁴Pa.s (0.5 centipoise) so that the second fluid is not capable of significantly penetrating into the pores during the process of agitation. Examples of the second fluid include but are not limited to saturated hydrocarbons or alkanes, such as hexane, heptane, and octane.

The prosthesis is rinsed in a third fluid subsequent to the application of the first solvent or the agitation of the prosthesis. The third fluid typically has a low capillary permeation, corresponding to a contact angle greater than about 90°. The third fluid has a viscosity of not less than 0,001 Pa.s (1.0 centipoise). The third fluid is not capable of significantly penetrating into the pores during the rising stage. In one embodiment, the third fluid is capable of significantly dissolving both the therapeutic substance and the polymeric material. Rinsing is conducted rapidly for example for a duration in a range from 1 second to 15 seconds. The specific duration depends on the solubility of the therapeutic substance and the polymeric material characteristics. The rinsing is repeated, if desired, until all traces of therapeutic substance and polymeric material are removed from the stent surface.

In an alternative embodiment, the third fluid is capable of significantly dissolving the therapeutic substance but not the polymeric material. As a result, traces of therapeutic substance are removed from the surface of the prosthesis, leaving a polymeric coating covering the surface of the prosthesis including the pores. The polymeric coating serves as an additional rate-reducing membrane. Useful examples of third fluid include but are not limited to DMSO, water, DMSO in an aqueous solution, and glycerol. The third fluid can be removed from the body of the prosthesis using a technique such as evaporation in ambient pressure, room temperature and anhydrous atmosphere and/or by exposure to mild heat (e.g., 60° C) under vacuum condition. The first, second, third fluids, alone or in conjunction with the polymeric material should not adversely affect the characteristics and composition of the therapeutic substance.

In another example, a polymeric material, such as a material capable of swell-loading or post-loading, can be deposited into the pores. Swell-loading occurs when a polymeric carrier is soaked with a therapeutic substance/solvent solution. The polymer swells, receiving the therapeutic substance in the polymer matrices. Once the solvent is removed, the polymer collapses and is impregnated with the therapeutic substance. Examples of polymeric material that are susceptible to swell-loading include thermoplastic polymers such as polyurethanes, polylactic acid, and polyglycolic acid, and non-thermoplastic polymers such as polyethyleneglycol, polyvinyl alcohol, polyacrylamide, and tecophilic polymers. The method of depositing a polymeric material into the pores is generally similar to the above-described methods with an addition of a curing step for the non-thermoplastic polymers subsequent to the rinsing step. One of ordinary skill in the art of polymer fabrication understands how to cure a non-thermoplastic polymer.

In another example, a radiopaque substance such as gold is deposited into the pores. The process of depositing radioactive isotopes is generally similar to the methods described above with a radiopaque substance dispersed and suspended in fine particles through a first fluid. The first fluid can have a high capillary permeation or a contact angle not higher than about 90°. The first fluid has a viscosity not higher than about ten centipoise and can be volatile, ensuring that the first fluid evaporates more readily and easily. Useful examples of first fluids include acetone, ethanol, methanol, isopropanol, tetrahydrofuran, and ethyl acetate.

The first fluid is applied to a porous prosthesis for a predetermined period of time, typically about 30 minutes. After application, the first solvent is removed from the prosthesis using a technique such as evaporation in ambient pressure, room temperature, and anhydrous atmosphere and/or by exposure to mild heat (for example, 60° C) under vacuum condition. The prosthesis may have a gross formation of radiopaque substance gathered on the prosthesis body surface. The radiopaque formation can be removed by immersing the prosthesis in a second fluid and agitating the prosthesis via mechanical perturbation techniques, such as vortexing or vigorous shaking. The second fluid can have a low capillary permeation or a contact angle greater than about 90° and a viscosity not less than about 5.10⁻⁴ Pa.s (0.5 centipoise) so that the second fluid is not capable of significantly penetrating into the pores during the process of agitation. Examples of the second fluid include but are not limited to saturated hydrocarbons or alkanes, such as hexane, heptane, and octane.

The prosthesis is rinsed in a third fluid subsequent to the application of the first fluid or the agitation of the prosthesis. The third fluid has a low capillary permeation or a contact angle greater than about 90°, and has a viscosity not less than about 0,001 Pa.s (1.0 centipoise) so that the third fluid is not capable of significantly penetrating into the pores during the rinsing stage. The rinsing is conducted rapidly, for example in a range from 1 second to 15 seconds, since an extended exposure duration may result in penetration of the third fluid into the pores. Suitable materials for the third fluid include DMSO, water, glyme and glycerol. Rinsing is repeated, if desired, until all traces of radiopaque substance are removed from the surface of the stent. The third fluid is removed from the body of the prosthesis using a technique such as evaporation in ambient pressure, room temperature and anhydrous atmosphere or by exposure to mild heat (e.g., 60° C) under vacuum condition. Sintering of the radiopaque material deposited in the pores is performed to bond particles of the radiopaque material without melting the particles. Appropriate pressure and temperature of radiopaque material sintering is specific to the particular material in a manner well known to one having ordinary skill in the art.

Several examples illustrate various methods for depositing substances such as therapeutic substances on a stent. The examples illustrate but do not limit the possible techniques for depositing substances.

### EXAMPLE 1

Trapidil is dissolved in ethanol by conventional methods. Trapidil makes up about 15% by weight of the total weight of the solution. A stent having a porous surface is immersed in the solution for 30 minutes. The stent is removed and mounted on a mandrel at ambient pressure, room temperature, and anhydrous atmosphere for approximately 30 minutes, until the ethanol is evaporated. The stent is submerged in hexane, followed by mechanical perturbation in a vortex apparatus for about 15 seconds. The stent is removed from the non-solvent and rinsed with water for about 5 seconds. The stent is dried at ambient pressure, room temperature, and anhydrous atmosphere.

### EXAMPLE 2

Trapidil is dissolved in ethanol by conventional methods. Trapidil makes up about 20% by weight of the total weight of the solution. A stent having a porous surface is immersed in the solution for 20 minutes. The stent is removed and mounted on a mandrel at ambient pressure, room temperature, and anhydrous atmosphere for approximately 30 minutes, until the ethanol is evaporated. The stent is submerged in heptane, followed by mechanical perturbation in vortex apparatus for 45 seconds. The stent is removed from the non-solvent and rinsed with dimethylsulfoxide (DMSO) for about 5 seconds. After rinsing, the mandrel is placed in ambient pressure, room temperature, and anhydrous atmosphere for approximately 2 hours, until the DMSO is evaporated.

### EXAMPLE 3

Trapidil is dissolved in ethanol by conventional methods. Trapidil makes up about 25% by weight of the total weight of the solution. A stent having a porous surface is immersed in the solution for 20 minutes. The stent is removed and mounted on a mandrel at ambient pressure, room temperature, and anhydrous atmosphere for approximately 30 minutes, until the ethanol is evaporated. The stent is submerged in heptane, followed by mechanical perturbation in vortex apparatus for about 60. The stent is removed from the non-solvent and rinsed with a dimethylsulfoxide (DMSO) aqueous solution (having a 1:1 DMSO-water ratio) for about 5 seconds. After rinsing, the mandrel is placed in ambient pressure, room temperature, and anhydrous atmosphere for approximately 2 hours. Next, the prosthesis was placed in an oven under vacuum condition and at a temperature of about 60° C for 24 hours, until all of the DMSO and water evaporated.

### EXAMPLE 4

Trapidil is dissolved in ethanol by conventional methods. Trapidil makes up about 25% by weight of the total weight of the solution. A stent having a porous surface is immersed in the solution for 30 minutes. The stent is removed and mounted on a mandrel at ambient pressure, room temperature, and anhydrous conditions for approximately 30 minutes, until the ethanol is evaporated. The stent is submerged in heptane, followed by mechanical perturbation in a vortex apparatus for about 45 seconds. The stent is removed from the non-solvent and rinsed, for about 10 seconds, with solution of ethylene vinyl alcohol and DMSO, the ethylene vinyl alcohol constituting about 1% by weight of the total weight of the solution. The mandrel is placed in ambient pressure, room temperature, and anhydrous condition for approximately 2 hours. The stent is placed in a oven under vacuum condition and at a temperature of about 60° C for 24 hours, until all of the DMSO is evaporated. A polymeric coating remains on the surface of the stent.

### EXAMPLE 5

Trapidil is dissolved in ethanol by conventional methods. Trapidil makes up about 20% by weight of the total weight of the solution. A stent having a porous surface is immersed in the solution for 40 minutes. The stent is removed and mounted on a mandrel at ambient pressure, room temperature, and anhydrous conditions for approximately 30 minutes, until the ethanol is evaporated. The stent is submerged in heptane, followed by mechanical perturbation in a vortex apparatus for about 30 seconds. The stent is removed from the non-solvent and rinsed, for about 10 seconds, with a solution containing ethylene vinyl alcohol, trapidil, and DMSO. The ethylene vinyl alcohol constitutes about 1% by weight and the trapidil constitutes about 0.33% by weight of the total weight of the solution. The mandrel is placed in ambient pressure, room temperature, and anhydrous condition for approximately 2 hours. The stent is placed in a oven under vacuum condition and at a temperature of about 60° C for 24 hours, until all of the DMSO is evaporated. A polymeric coating having a trapidil impregnated therein remains on the stent.

### EXAMPLE 6

Trapidil was dissolved in ethanol by conventional methods. Trapidil was used to make 40% by weight of the total weight of the solution. A stent having a porous surface was mounted on a mandrel and dipped in the 40% solution for 40 seconds. The stent was removed from the other end and dried at ambient pressure, room temperature, and anhydrous conditions for about 30 minutes, until the ethanol was evaporated. The stent was mounted on a mandrel again and rinsed in a heparin (DuraFlo™)/ Trapidil solution for 3 seconds. The solution constituted 0.6% by weight of Heparin and 0.6% by weight of Trapidil. The solvent used was Freon (Xerosolv) and n-Propanol in the ratio of 5:1 by volume. The stent was placed in a humidity controlled chamber at room temperature for 12 hours until all of the Freon and n-Propanol solvent system evaporated. A DuraFlo coating having Trapidil impregnated therein remained on the stent surface and inside the pores.

## Claims

1. A method of loading a substance into pores of an implantable prosthesis, the method comprising:
providing a prosthesis having a surface and pores formed in said surface;
applying to said prosthesis a first fluid having an added substance, wherein during said act of applying said first fluid penetrates into said pores;
removing said first fluid from said prosthesis substantially to elimination;
applying to said prosthesis a second fluid to remove said substance from said surface of said prosthesis, said second fluid having a contact angle greater than about 90° so as to prevent said second fluid from significantly penetrating into said pores to remove said substance out from said pores during the application of said second fluid; and
removing said second fluid from said prosthesis substantially to elimination,
wherein said substance is deposited into said pores.

2. The method according to Claim 1, wherein said acts of removing said first fluid and said second fluid comprise evaporating said first fluid and said second fluid from said prosthesis.

3. The method according to Claim 1, additionally comprising prior to said act of applying said second fluid:
immersing said prosthesis in a third fluid, wherein said substance does not significantly dissolve in said third fluid;
agitating said prosthesis in said third fluid to significantly remove said substance from said surface of said prosthesis, wherein said substance remains in said pores; and
removing said prosthesis from said third fluid.

4. The method according to Claim 3, wherein said third fluid has a contact angle greater than about 90°.

5. The method according to Claim 1, wherein said substance significantly dissolves when in contact with said second fluid.

6. The method according to Claim 1, wherein said substance is a first therapeutic substance.

7. The method according to Claim 6, wherein said first therapeutic substance significantly dissolves when in contact with said second fluid.

8. The method according to Claim 6, wherein said second fluid has a boiling point of not greater than about 60°.

9. The method according to Claim 6, additionally comprising, prior to said act of applying said second fluid:
immersing said prosthesis in a third fluid, wherein said first therapeutic substance does not significantly dissolve in said third fluid;
agitating said prosthesis in said third fluid to significantly remove said first therapeutic substance from said surface of said prosthesis, wherein said first therapeutic substance remains in said pores; and
removing said prosthesis from said third fluid.

10. The method according to Claim 9, wherein said third fluid has a contact angle greater than about 90°.

11. The method according to Claim 6, wherein said second fluid comprises a second therapeutic substance added thereto, such that after said act of significantly removing said second fluid from said prosthesis, a coating of said second therapeutic substance remains on said surface of said prosthesis.

12. The method according to Claim 11, wherein said second therapeutic substance is different than said first therapeutic substance.

13. The method according to Claim 6, wherein said second fluid comprises a polymeric material added thereto, such that after said act of significantly removing said second fluid from said prosthesis, a coating of said polymeric material remains on said surface of said prosthesis.

14. The method according to Claim 6, wherein said second fluid comprises a combination of a polymeric material and a second therapeutic substance added thereto, such that after said act of significantly removing said second fluid from said prosthesis, a coating of said polymeric material containing said second therapeutic substance remains on said surface of said prosthesis.

15. The method according to Claim 1, wherein said substance is a polymeric material.

16. The method according to Claim 15, wherein said polymeric material significantly dissolves when in contact with said second fluid.

17. The method according to Claim 15, additionally comprising, prior to said act of applying said second fluid:
immersing said prosthesis in a third fluid, wherein said polymeric material does not significantly dissolve in said third fluid;
agitating said prosthesis in said third fluid to significantly remove said polymeric material from said surface of said prosthesis, wherein said polymeric material remains in said pores; and
removing said prosthesis from said third fluid.

18. The method according to Claim 17, wherein said third fluid has a contact angle greater than about 90°.

19. The method according to Claim 15, wherein said polymeric material is a non-thermoplastic polymer and the method additionally comprises the act of curing said non-thermoplastic polymer deposited in said pores.

20. The method according to Claim 1, wherein said substance is a combination of a polymeric material and a therapeutic substance.

21. The method according to Claim 20, additionally comprising, prior to said act of applying said second fluid:
immersing said prosthesis in a third fluid, wherein said polymeric material and said therapeutic substance do not significantly dissolve in said third fluid;
agitating said prosthesis in said third fluid to significantly remove said polymeric material and said therapeutic substance from said surface of said prosthesis, wherein said polymeric material containing said therapeutic substance remains in said pores; and
removing said prosthesis from said third fluid.

22. The method according to Claim 21, wherein said third fluid has a contact angle greater than about 90°.

23. The method according to Claim 20, wherein said polymeric material and said therapeutic substance dissolves when in contact with said second fluid such that any of said polymeric material and said therapeutic substance disposed on said surface of said prosthesis are significantly removed.

24. The method according to Claim 20, wherein said therapeutic substance dissolves when in contact with said second fluid, wherein after said act of significantly removing said second fluid a coating made from said polymeric material remains on said surface of said prosthesis and covers said pores.

25. The method according to Claim 1, wherein said substance is a radioactive isotope.

26. The method according to Claim 25, additionally comprising, prior to said act of applying said second fluid:
immersing said prosthesis in a third fluid;
agitating said prosthesis in said third fluid to significantly remove said radioactive isotope from said surface of said prosthesis; and
removing said prosthesis from said third fluid.

27. The method according to Claim 26, wherein said third fluid has a contact angle greater than about 90°.

28. The method according to Claim 1, wherein said substance is a radiopaque material.

29. The method according to Claim 28, additionally comprising, subsequent to said act of significantly removing said second fluid, sintering said radiopaque material deposited in said pores.

30. The method according to Claim 28, additionally comprising, prior to said act of applying said second fluid:
immersing said prosthesis in a third fluid;
agitating said prosthesis in said third fluid to significantly remove said radiopaque material from said surface of said prosthesis; and
removing said prosthesis from said third fluid.

31. The method according to Claim 30, wherein said third fluid has a contact angle greater than about 90° .

32. The method according to Claim 1, wherein applying said second fluid to said prosthesis comprises immersing said prosthesis in said second fluid, wherein said substance does not significantly dissolve in said second fluid.

33. The method according to Claim 32, further comprising agitating said prosthesis in said second fluid to significantly remove said substance from a surface of said body;
removing said prosthesis from said second fluid;
rinsing said prosthesis with a third fluid, wherein during said act of rinsing, said third fluid is not capable of significantly penetrating into said pores; and
significantly removing said third fluid from said prosthesis, wherein said substance is deposited in said pores.

34. The method according to Claim 33, wherein said first fluid has a contact angle less than about 90°.

35. The method according to claim 33, wherein said second fluid has a lower capillary permeation than said first fluid.

36. The method according to Claim 33, wherein said substance dissolves when in contact with said third fluid.

37. The method according to Claim 33, wherein said third fluid has a lower capillary permeation than said first fluid.

38. The method according to Claim 33, wherein said third fluid has a contact angle greater than about 90°.

39. The method according to Claim 1, wherein providing said prosthesis having said surface and pores formed in said surface comprises forming said pores in preselected locations of a body of said implantable prosthesis.

40. The method according to Claim 39, wherein providing said prosthesis having said surface and pores formed in said surface further comprises:
forming a structure of said body having a generally cylindrical shape, an outer surface capable of contacting an inner lumen surface of a passageway, and a hollow bore which extends longitudinally through said body structure to define an inner surface, said body structure having a thickness.

41. The method according to Claim 39, further comprising:
controlling formation of the pores to a predetermined depth.

42. The method according to Claim 39 further comprising:
ontrolling formation of the pores to a predetermined geometrical shape.

43. An implantable prosthesis, comprising:
a body structure having a generally cylindrical shape, an outer surface capable of contacting an inner lumen surface of a passageway, and a hollow bore which extends longitudinally through said body structure to define an inner surface, said body structure having a thickness;
said body structure having a plurality of depots formed on at least a region of said outer surface, said depots having a depth less than said thickness of said body structure, and said depots having a preselected and controlled location and a preselected and controlled depth.

44. The implantable prosthesis of Claim 43, wherein said prosthesis is a stent.

45. The implantable prosthesis of Claim 43, wherein said depots are laser trenches formed by exposing said surface to an energy discharge from a laser.

46. The implantable prosthesis of Claim 43, wherein said preselected and controlled depth is equal to 10% to 90% of said thickness.

47. The implantable prosthesis of Claim 43, wherein said preselected and controlled depth is not greater than about 65% of said thickness.

48. The implantable prosthesis of Claim 43, wherein said depots have a preselected shape.

49. The implantable prosthesis of Claim 48, wherein said preselected depot shape is generally cylindrical.

50. The implantable prosthesis of Claim 48, wherein said preselected depot shape is generally conical.

51. The implantable prosthesis of Claim 50, wherein said conical shaped depot has an open end and a closed end, said open end contacts said passageway inner lumen surface, and said open end has a diameter smaller than a diameter of said closed end.

52. The implantable prosthesis of Claim 43, further comprising a substance deposited into said depots.

53. The implantable prosthesis of Claim 43, further comprising a therapeutic substance deposited into said depots.

54. The implantable prosthesis of Claim 43, further comprising a therapeutic substance deposited into said depots, wherein said therapeutic substance is selected from a group of antineoplastic, antiplatelet, anticoagulant, fribrinolytics, antimitotic, thrombin inhibitor, antiinflammatory, and antiproliferative substances.

55. The implantable prosthesis of Claim 43, further comprising a therapeutic substance deposited into said depots, wherein said therapeutic substance is a radioactive isotope.

56. The implantable prosthesis of Claim 43, further comprising a substance deposited into said depots, wherein said substance is a polymeric material.

57. The implantable prosthesis of Claim 43, further comprising a substance deposited into said depots, wherein said substance is a polymeric material containing a therapeutic substance.

58. The implantable prosthesis of Claim 43, further comprising a substance deposited into said depots, wherein said substance is a polymeric material containing a therapeutic substance, said therapeutic substance is selected from a group of antineoplastic, antiplatelet, anticoagulant, fibrinolytic, antimitotic, thrombin inhibitor, antiinflammatory, and antiproliferative substances.

59. The implantable prosthesis of Claim 43, further comprising a substance deposited into said depots, wherein said substance is a radiopaque substance.

60. The implantable prosthesis of Claim 43, further comprising a first therapeutic substance deposited in said depots and a polymeric coating formed on said lumen contacting surface of said body, wherein said polymeric coating covers said depots and reduces the rate at which said first therapeutic substance is released from said depots.

61. The implantable prosthesis of Claim 60, wherein said polymeric coating contains a second therapeutic substance.

## Patentansprüche

1. Verfahren zum Einbringen einer Substanz in Poren einer implantierbaren Prothese, wobei das Verfahren umfasst:
Bereitstellen einer Prothese mit einer Oberfläche und in der Oberfläche ausgebildeten Poren,
Aufbringen eines ersten Fluids, das eine zugesetzte Substanz aufweist, auf die Prothese, wobei das erste Fluid während des Vorgangs des Aufbringens in die Poren eindringt,
Entfernen des ersten Fluids von der Prothese, um es im Wesentlichen zu beseitigen,
Aufbringen eines zweiten Fluids auf die Prothese zur Entfernung der Substanz von der Oberfläche der Prothese, wobei das zweite Fluid einen Kontaktwinkel von mehr als etwa 90° aufweist, so dass verhindert wird, dass das zweite Fluid signifikant in die Poren eindringt und die Substanz von den Poren während des Aufbringens des zweiten Fluids entfernt, und
Entfernen des zweiten Fluids von der Prothese, um es im Wesentlichen zu beseitigen,
wobei die Substanz in den Poren abgeschieden wird.

2. Verfahren nach Anspruch 1, wobei die Vorgänge des Entfernens des ersten Fluids und des zweiten Fluids das Verdampfen des ersten Fluids und des zweiten Fluids von der Prothese umfassen.

3. Verfahren nach Anspruch 1, das vor dem Vorgang des Aufbringens des zweiten Fluids zusätzlich umfasst:
Eintauchen der Prothese in ein drittes Fluid, wobei sich die Substanz nicht signifikant in dem dritten Fluid löst,
Bewegen der Prothese in dem dritten Fluid zur signifikanten Entfernung der Substanz von der Oberfläche der Prothese, wobei die Substanz in den Poren verbleibt, und
Entfernen der Prothese aus dem dritten Fluid.

4. Verfahren nach Anspruch 3, bei dem das dritte Fluid einen Kontaktwinkel von mehr als etwa 90° aufweist.

5. Verfahren nach Anspruch 1, bei dem sich die Substanz signifikant löst, wenn sie mit dem zweiten Fluid in Kontakt ist.

6. Verfahren nach Anspruch 1, bei dem die Substanz eine erste therapeutische Substanz ist.

7. Verfahren nach Anspruch 6, bei dem sich die erste therapeutische Substanz signifikant löst, wenn sie mit dem zweiten Fluid in Kontakt ist.

8. Verfahren nach Anspruch 6, bei dem das zweite Fluid einen Siedepunkt von nicht mehr als etwa 60° aufweist.

9. Verfahren nach Anspruch 6, das vor dem Vorgang des Aufbringens des zweiten Fluids zusätzlich umfasst:
Eintauchen der Prothese in ein drittes Fluid, wobei sich die erste therapeutische Substanz nicht signifikant in dem dritten Fluid löst,
Bewegen der Prothese in dem dritten Fluid zur signifikanten Entfernung der ersten therapeutischen Substanz von der Oberfläche der Prothese, wobei die erste therapeutische Substanz in den Poren verbleibt, und
Entfernen der Prothese aus dem dritten Fluid.

10. Verfahren nach Anspruch 9, bei dem das dritte Fluid einen Kontaktwinkel von mehr als etwa 90° aufweist.

11. Verfahren nach Anspruch 6, bei dem das zweite Fluid eine diesem zugesetzte zweite therapeutische Substanz umfasst, und zwar derart, dass nach dem Vorgang des signifikanten Entfernens des zweiten Fluids von der Prothese eine Beschichtung der zweiten therapeutischen Substanz auf der Oberfläche der Prothese verbleibt.

12. Verfahren nach Anspruch 11, bei dem die zweite therapeutische Substanz von der ersten therapeutischen Substanz verschieden ist.

13. Verfahren nach Anspruch 6, bei dem das zweite Fluid ein diesem zugesetztes polymeres Material umfasst, und zwar derart, dass nach dem Vorgang des signifikanten Entfernens des zweiten Fluids von der Prothese eine Beschichtung des polymeren Materials auf der Oberfläche der Prothese verbleibt.

14. Verfahren nach Anspruch 6, bei dem das zweite Fluid eine Kombination aus einem polymeren Material und einer diesem zugesetzten zweiten therapeutischen Substanz umfasst, und zwar derart, dass nach dem Vorgang des signifikanten Entfernens des zweiten Fluids von der Prothese eine Beschichtung des polymeren Materials, das die zweite therapeutische Substanz enthält, auf der Oberfläche der Prothese verbleibt.

15. Verfahren nach Anspruch 1, bei dem die Substanz ein polymeres Material ist.

16. Verfahren nach Anspruch 15, bei dem sich das polymere Material signifikant löst, wenn es mit dem zweiten Fluid in Kontakt ist.

17. Verfahren nach Anspruch 15, das vor dem Vorgang des Aufbringens des zweiten Fluids zusätzlich umfasst:
Eintauchen der Prothese in ein drittes Fluid, wobei sich das polymere Material nicht signifikant in dem dritten Fluid löst,
Bewegen der Prothese in dem dritten Fluid zur signifikanten Entfernung des polymeren Materials von der Oberfläche der Prothese, wobei das polymere Material in den Poren verbleibt, und
Entfernen der Prothese aus dem dritten Fluid.

18. Verfahren nach Anspruch 17, bei dem das dritte Fluid einen Kontaktwinkel von mehr als etwa 90° aufweist.

19. Verfahren nach Anspruch 15, bei dem das polymere Material ein nichtthermoplastisches Polymer ist und das Verfahren zusätzlich den Vorgang des Härtens des nicht-thermoplastischen Polymers, das in den Poren abgeschieden ist, umfasst.

20. Verfahren nach Anspruch 1, bei dem die Substanz eine Kombination aus einem polymeren Material und einer therapeutischen Substanz ist.

21. Verfahren nach Anspruch 20, das vor dem Vorgang des Aufbringens des zweiten Fluids zusätzlich umfasst:
Eintauchen der Prothese in ein drittes Fluid, wobei sich das polymere Material und die therapeutische Substanz nicht signifikant in dem dritten Fluid lösen,
Bewegen der Prothese in dem dritten Fluid zur signifikanten Entfernung des polymeren Materials und der therapeutischen Substanz von der Oberfläche der Prothese, wobei das polymere Material, das die therapeutische Substanz enthält, in den Poren verbleibt, und
Entfernen der Prothese aus dem dritten Fluid.

22. Verfahren nach Anspruch 21, bei dem das dritte Fluid einen Kontaktwinkel von mehr als etwa 90° aufweist.

23. Verfahren nach Anspruch 20, bei dem sich das polymere Material und die therapeutische Substanz lösen, wenn sie mit dem zweiten Fluid in Kontakt sind, so dass jedwedes des polymeren Materials und der therapeutischen Substanz, die auf der Oberfläche der Prothese abgeschieden sind, signifikant entfernt wird.

24. Verfahren nach Anspruch 20, bei dem sich die therapeutische Substanz löst, wenn sie mit dem zweiten Fluid in Kontakt ist, wobei nach dem Vorgang des signifikanten Entfernens des zweiten Fluids eine aus dem polymeren Material gebildete Beschichtung auf der Oberfläche der Prothese verbleibt und die Poren bedeckt.

25. Verfahren nach Anspruch 1, bei dem die Substanz ein radioaktives Isotop ist.

26. Verfahren nach Anspruch 25, das vor dem Vorgang des Aufbringens des zweiten Fluids zusätzlich umfasst:
Eintauchen der Prothese in ein drittes Fluid,
Bewegen der Prothese in dem dritten Fluid zur signifikanten Entfernung des radioaktiven Isotops von der Oberfläche der Prothese, und
Entfernen der Prothese aus dem dritten Fluid.

27. Verfahren nach Anspruch 26, bei dem das dritte Fluid einen Kontaktwinkel von mehr als etwa 90° aufweist.

28. Verfahren nach Anspruch 1, bei dem die Substanz ein strahlenundurchlässiges Material ist.

29. Verfahren nach Anspruch 28, das anschließend an den Vorgang des signifikanten Entfernens des zweiten Fluids zusätzlich das Sintern des in den Poren abgeschiedenen strahlenundurchlässigen Materials umfasst.

30. Verfahren nach Anspruch 28, das vor dem Vorgang des Aufbringens des zweiten Fluids zusätzlich umfasst:
Eintauchen der Prothese in ein drittes Fluid,
Bewegen der Prothese in dem dritten Fluid zur signifikanten Entfernung des strahlenundurchlässigen Materials von der Oberfläche der Prothese, und
Entfernen der Prothese aus dem dritten Fluid.

31. Verfahren nach Anspruch 30, bei dem das dritte Fluid einen Kontaktwinkel von mehr als etwa 90° aufweist.

32. Verfahren nach Anspruch 1, bei dem das Aufbringen des zweiten Fluids auf die Prothese das Eintauchen der Prothese in das zweite Fluid umfasst, wobei sich die Substanz nicht signifikant in dem zweiten Fluid löst.

33. Verfahren nach Anspruch 32, das ferner umfasst:
Bewegen der Prothese in dem zweiten Fluid zur signifikanten Entfernung der Substanz von einer Oberfläche des Körpers,
Entfernen der Prothese aus dem zweiten Fluid,
Spülen der Prothese mit einem dritten Fluid, wobei das dritte Fluid während des Vorgangs des Spülens nicht signifikant in die Poren eindringen kann, und
signifikantes Entfernen des dritten Fluids von der Prothese, wobei die Substanz in den Poren abgeschieden wird.

34. Verfahren nach Anspruch 33, bei dem das erste Fluid einen Kontaktwinkel von weniger als etwa 90° aufweist.

35. Verfahren nach Anspruch 33, bei dem das zweite Fluid eine geringere Kapillarpermeation als das erste Fluid aufweist.

36. Verfahren nach Anspruch 33, bei dem sich die Substanz löst, wenn sie mit dem dritten Fluid in Kontakt ist.

37. Verfahren nach Anspruch 33, bei dem das dritte Fluid eine geringere Kapillarpermeation als das erste Fluid aufweist.

38. Verfahren nach Anspruch 33, bei dem das dritte Fluid einen Kontaktwinkel von mehr als etwa 90° aufweist.

39. Verfahren nach Anspruch 1, bei dem das Bereitstellen der Prothese, welche die Oberfläche und die in der Oberfläche ausgebildeten Poren umfasst, das Bilden der Poren in vorausgewählten Stellen eines Körpers der implantierbaren Prothese umfasst.

40. Verfahren nach Anspruch 39, bei dem das Bereitstellen der Prothese, welche die Oberfläche und die in der Oberfläche ausgebildeten Poren aufweist, ferner umfasst:
Bilden einer Struktur des Körpers mit einer im Allgemeinen zylindrischen Form, einer Außenoberfläche, die mit einer Innenlumenoberfläche eines Durchgangs in Kontakt kommen kann, und einer hohlen Bohrung, die sich in Längsrichtung durch die Körperstruktur erstreckt, so dass eine Innenoberfläche definiert wird, wobei die Körperstruktur eine Dicke aufweist.

41. Verfahren nach Anspruch 39, das ferner das Steuern der Bildung der Poren zu einer vorgegebenen Tiefe umfasst.

42. Verfahren nach Anspruch 39, das ferner das Steuern der Bildung der Poren zu einer vorgegebenen geometrischen Form umfasst.

43. Implantierbare Prothese, umfassend:
eine Körperstruktur mit einer im Allgemeinen zylindrischen Form, einer Außenoberfläche, die mit einer Innenlumenoberfläche eines Durchgangs in Kontakt kommen kann, und einer hohlen Bohrung, die sich in Längsrichtung durch die Körperstruktur erstreckt, so dass eine Innenoberfläche definiert wird, wobei die Körperstruktur eine Dicke aufweist,
wobei die Körperstruktur eine Mehrzahl von Depots umfasst, die auf mindestens einem Bereich der Außenoberfläche ausgebildet sind, wobei die Depots eine Tiefe aufweisen, die geringer ist als die Dicke der Körperstruktur, und wobei die Depots an einer vorausgewählten und gesteuerten Stelle vorliegen und eine vorausgewählte und gesteuerte Tiefe aufweisen.

44. Implantierbare Prothese nach Anspruch 43, wobei die Prothese ein Stent ist.

45. Implantierbare Prothese nach Anspruch 43, bei der die Depots Lasergräben sind, die durch Aussetzen der Oberfläche einer Energieentladung von einem Laser gebildet worden sind.

46. Implantierbare Prothese nach Anspruch 43, bei der die vorausgewählte und gesteuerte Tiefe gleich 10 % bis 90 % der Dicke beträgt.

47. Implantierbare Prothese nach Anspruch 43, bei der die vorausgewählte und gesteuerte Tiefe nicht mehr als etwa 65 % der Dicke beträgt.

48. Implantierbare Prothese nach Anspruch 43, bei der die Depots eine vorausgewählte Form aufweisen.

49. Implantierbare Prothese nach Anspruch 48, bei der die vorausgewählte Depotform im Allgemeinen zylindrisch ist.

50. Implantierbare Prothese nach Anspruch 48, bei der die vorausgewählte Depotform im Allgemeinen konisch ist.

51. Implantierbare Prothese nach Anspruch 50, bei der das konisch geformte Depot ein offenes Ende und ein geschlossenes Ende aufweist, wobei das offene Ende die Durchgangsinnenlumenoberfläche kontaktiert und das offene Ende einen Durchmesser aufweist, der kleiner ist als der Durchmesser des geschlossenen Endes.

52. Implantierbare Prothese nach Anspruch 43, die ferner eine in den Depots abgeschiedene Substanz umfasst.

53. Implantierbare Prothese nach Anspruch 43, die ferner eine in den Depots abgeschiedene therapeutische Substanz umfasst.

54. Implantierbare Prothese nach Anspruch 43, die ferner eine in den Depots abgeschiedene therapeutische Substanz umfasst, wobei die therapeutische Substanz aus einer Gruppe von antineoplastischen, Antiplättchen-, gerinnungshemmenden, fibrinolytischen, antimitotischen, Thrombininhibitor-, entzündungshemmenden und antiproliferativen Substanzen ausgewählt ist.

55. Implantierbare Prothese nach Anspruch 43, die ferner eine in den Depots abgeschiedene therapeutische Substanz umfasst, wobei die therapeutische Substanz ein radioaktives Isotop ist.

56. Implantierbare Prothese nach Anspruch 43, die ferner eine in den Depots abgeschiedene Substanz umfasst, wobei die Substanz ein polymeres Material ist.

57. Implantierbare Prothese nach Anspruch 43, die ferner eine in den Depots abgeschiedene Substanz umfasst, wobei die Substanz ein polymeres Material ist, das eine therapeutische Substanz enthält.

58. Implantierbare Prothese nach Anspruch 43, die ferner eine in den Depots abgeschiedene Substanz umfasst, wobei die Substanz ein polymeres Material ist, das eine therapeutische Substanz enthält, wobei die therapeutische Substanz aus einer Gruppe von antineoplastischen, Antiplättchen-, gerinnungshemmenden, fibrinolytischen, antimitotischen, Thrombininhibitor-, entzündungshemmenden und antiproliferativen Substanzen ausgewählt ist.

59. Implantierbare Prothese nach Anspruch 43, die ferner eine in den Depots abgeschiedene Substanz umfasst, wobei die Substanz eine strahlenundurchlässige Substanz ist.

60. Implantierbare Prothese nach Anspruch 43, die ferner eine in den Depots abgeschiedene erste therapeutische Substanz und eine polymere Beschichtung, die auf der Lumenkontaktierenden Oberfläche des Körpers ausgebildet ist, umfasst, wobei die polymere Beschichtung die Depots bedeckt und die Rate vermindert, mit der die erste therapeutische Substanz aus den Depots freigesetzt wird.

61. Implantierbare Prothese nach Anspruch 60, bei der die polymere Beschichtung eine zweite therapeutische Substanz enthält.

## Revendications

1. Procédé servant à introduire une substance dans des pores d'une prothèse implantable, lequel procédé comporte :
- le fait de prendre une prothèse présentant une surface et des pores formés dans cette surface,
- le fait d'appliquer sur cette prothèse un premier fluide contenant une substance ajoutée, lequel premier fluide pénètre dans lesdits pores pendant ladite opération d'application,
- le fait d'enlever ce premier fluide de ladite prothèse, pratiquement jusqu'à l'en éliminer,
- le fait d'appliquer sur ladite prothèse un deuxième fluide, afin d'enlever ladite substance de ladite surface de la prothèse, lequel deuxième fluide présente un angle de contact supérieur à environ 90°, ce qui empêche ce deuxième fluide de pénétrer en quantité significative dans lesdits pores et d'enlever de ces pores ladite substance pendant l'application dudit deuxième fluide ;
- et le fait d'enlever ce deuxième fluide de ladite prothèse, pratiquement jusqu'à l'en éliminer,
grâce à quoi ladite substance se trouve déposée dans lesdits pores.

2. Procédé conforme à la revendication 1, dans lequel lesdites opérations d'enlèvement du premier fluide et du deuxième fluide comprennent le fait de chasser de ladite prothèse le premier fluide et le deuxième fluide par évaporation.

3. Procédé conforme à la revendication 1, qui comporte en outre, avant ladite opération d'application du deuxième fluide :
- le fait de plonger ladite prothèse dans un troisième fluide dans lequel ladite substance ne se dissout pas en quantité significative,
- le fait d'agiter ladite prothèse dans ce troisième fluide, afin d'enlever en quantité significative ladite substance de ladite surface de la prothèse, tandis que ladite substance reste dans lesdits pores,
- et le fait d'enlever ladite prothèse de ce troisième fluide.

4. Procédé conforme à la revendication 3, dans lequel ledit troisième fluide présente un angle de contact supérieur à environ 90°.

5. Procédé conforme à la revendication 1, dans lequel ladite substance se dissout en quantité significative quand elle est en contact avec ledit deuxième fluide.

6. Procédé conforme à la revendication 1, dans lequel ladite substance est une première substance thérapeutique.

7. Procédé conforme à la revendication 6, dans lequel ladite première substance thérapeutique se dissout en quantité significative quand elle est en contact avec ledit deuxième fluide.

8. Procédé conforme à la revendication 6, dans lequel ledit deuxième fluide présente un point d'ébullition qui ne dépasse pas environ 60 °C.

9. Procédé conforme à la revendication 6, qui comporte en outre, avant ladite opération d'application du deuxième fluide :
- le fait de plonger ladite prothèse dans un troisième fluide dans lequel ladite première substance thérapeutique ne se dissout pas en quantité significative,
- le fait d'agiter ladite prothèse dans ce troisième fluide, afin d'enlever en quantité significative ladite première substance thérapeutique de ladite surface de la prothèse, tandis que ladite première substance thérapeutique reste dans lesdits pores,
- et le fait d'enlever ladite prothèse de ce troisième fluide.

10. Procédé conforme à la revendication 9, dans lequel ledit troisième fluide présente un angle de contact supérieur à environ 90°.

11. Procédé conforme à la revendication 6, dans lequel ledit deuxième fluide contient une deuxième substance thérapeutique qui y a été ajoutée, de sorte qu'après ladite opération où l'on enlève en quantité significative ce deuxième fluide de ladite prothèse, il reste sur ladite surface de cette prothèse une couche de ladite deuxième substance thérapeutique.

12. Procédé conforme à la revendication 11, dans lequel ladite deuxième substance thérapeutique est différente de ladite première substance thérapeutique.

13. Procédé conforme à la revendication 6, dans lequel ledit deuxième fluide contient un matériau polymère qui y a été ajouté, de sorte qu'après ladite opération où l'on enlève en quantité significative ce deuxième fluide de ladite prothèse, il reste sur ladite surface de cette prothèse une couche de ce matériau polymère.

14. Procédé conforme à la revendication 6, dans lequel ledit deuxième fluide contient une combinaison d'un matériau polymère et d'une deuxième substance thérapeutique qui y ont été ajoutés, de sorte qu'après ladite opération où l'on enlève en quantité significative ce deuxième fluide de ladite prothèse, il reste sur ladite surface de cette prothèse une couche de ce matériau polymère contenant ladite deuxième substance thérapeutique.

15. Procédé conforme à la revendication 1, dans lequel ladite substance est un matériau polymère.

16. Procédé conforme à la revendication 15, dans lequel ledit matériau polymère se dissout en quantité significative quand il est en contact avec ledit deuxième fluide.

17. Procédé conforme à la revendication 15, qui comporte en outre, avant ladite opération d'application du deuxième fluide :
- le fait de plonger ladite prothèse dans un troisième fluide dans lequel ledit matériau polymère ne se dissout pas en quantité significative,
- le fait d'agiter ladite prothèse dans ce troisième fluide, afin d'enlever en quantité significative ledit matériau polymère de ladite surface de la prothèse, tandis que ledit matériau polymère reste dans lesdits pores,
- et le fait d'enlever ladite prothèse de ce troisième fluide.

18. Procédé conforme à la revendication 17, dans lequel ledit troisième fluide présente un angle de contact supérieur à environ 90°.

19. Procédé conforme à la revendication 15, dans lequel ledit matériau polymère est un polymère non-thermoplastique, et lequel procédé comporte en outre le fait de faire durcir ce polymère non-thermoplastique déposé dans les pores.

20. Procédé conforme à la revendication 1, dans lequel ladite substance est une combinaison d'un matériau polymère et d'une substance thérapeutique.

21. Procédé conforme à la revendication 20, qui comporte en outre, avant ladite opération d'application du deuxième fluide :
- le fait de plonger ladite prothèse dans un troisième fluide dans lequel ledit matériau polymère et ladite substance thérapeutique ne se dissolvent pas en quantité significative,
- le fait d'agiter ladite prothèse dans ce troisième fluide, afin d'enlever en quantité significative ledit matériau polymère et ladite substance thérapeutique de ladite surface de la prothèse, tandis que ledit matériau polymère et ladite substance thérapeutique restent dans lesdits pores,
- et le fait d'enlever ladite prothèse de ce troisième fluide.

22. Procédé conforme à la revendication 21, dans lequel ledit troisième fluide présente un angle de contact supérieur à environ 90°.

23. Procédé conforme à la revendication 20, dans lequel ledit matériau polymère et ladite substance thérapeutique se dissolvent quand ils sont en contact avec ledit deuxième fluide, au point que tout dépôt de ces matériau polymère et substance thérapeutique présent sur ladite surface de ladite prothèse en est enlevé en quantité significative.

24. Procédé conforme à la revendication 20, dans lequel ladite substance thérapeutique se dissout quand elle est en contact avec ledit deuxième fluide, et dans lequel, après ladite opération où l'on enlève en quantité significative ce deuxième fluide, il reste sur ladite surface de cette prothèse une couche de ce matériau polymère qui recouvre lesdits pores.

25. Procédé conforme à la revendication 1, dans lequel ladite substance est un isotope radioactif.

26. Procédé conforme à la revendication 25, qui comporte en outre, avant ladite opération d'application du deuxième fluide :
- le fait de plonger ladite prothèse dans un troisième fluide,
- le fait d'agiter ladite prothèse dans ce troisième fluide, afin d'enlever en quantité significative ledit isotope radioactif de ladite surface de la prothèse,
- et le fait d'enlever ladite prothèse de ce troisième fluide.

27. Procédé conforme à la revendication 26, dans lequel ledit troisième fluide présente un angle de contact supérieur à environ 90°.

28. Procédé conforme à la revendication 1, dans lequel ladite substance est un produit radio-opaque.

29. Procédé conforme à la revendication 28, qui comporte en outre, après ladite opération où l'on enlève le deuxième fluide en quantité significative, le fait de fritter ledit produit radio-opaque déposé dans lesdits pores.

30. Procédé conforme à la revendication 28, qui comporte en outre, avant ladite opération d'application du deuxième fluide :
- le fait de plonger ladite prothèse dans un troisième fluide,
- le fait d'agiter ladite prothèse dans ce troisième fluide, afin d'enlever en quantité significative ledit produit radio-opaque de ladite surface de la prothèse,
- et le fait d'enlever ladite prothèse de ce troisième fluide.

31. Procédé conforme à la revendication 30, dans lequel ledit troisième fluide présente un angle de contact supérieur à environ 90°.

32. Procédé conforme à la revendication 1, dans lequel ladite opération d'application dudit deuxième fluide sur ladite prothèse comporte le fait de plonger cette prothèse dans ce deuxième fluide, et dans lequel ladite substance ne se dissout pas en quantité significative dans ce deuxième fluide.

33. Procédé conforme à la revendication 32, qui comporte en outre :
- le fait d'agiter ladite prothèse dans ledit deuxième fluide, afin d'enlever en quantité significative ladite substance d'une surface de ladite prothèse,
- le fait d'enlever ladite prothèse de ce deuxième fluide,
- le fait de rincer cette prothèse avec un troisième fluide qui ne peut pas, au cours de cette opération de rinçage, pénétrer en quantité significative dans lesdits pores,
- et le fait d'enlever en quantité significative ce troisième fluide de ladite prothèse,
ladite substance restant déposée dans lesdits pores.

34. Procédé conforme à la revendication 33, dans lequel ledit premier fluide présente un angle de contact inférieur à environ 90°.

35. Procédé conforme à la revendication 33, dans lequel ledit deuxième fluide présente un pouvoir mouillant plus faible que celui du premier fluide.

36. Procédé conforme à la revendication 33, dans lequel ladite substance se dissout quand elle est en contact avec ledit troisième fluide.

37. Procédé conforme à la revendication 33, dans lequel ledit troisième fluide présente un pouvoir mouillant plus faible que celui du premier fluide.

38. Procédé conforme à la revendication 33, dans lequel ledit troisième fluide présente un angle de contact supérieur à environ 90°.

39. Procédé conforme à la revendication 1, dans lequel le fait de prendre une prothèse présentant une surface et des pores formés dans cette surface comporte le fait de former lesdits pores en des points présélectionnés d'un corps de ladite prothèse implantable.

40. Procédé conforme à la revendication 39, dans lequel le fait de prendre une prothèse présentant une surface et des pores formés dans cette surface comporte en outre le fait de former une structure de corps de forme globalement cylindrique, présentant une surface externe qui peut entrer en contact avec une surface de lumière interne d'un conduit, ainsi qu'un évidement qui traverse ladite structure de corps dans sa longueur pour y définir une surface interne, cette structure de corps ayant une certaine épaisseur.

41. Procédé conforme à la revendication 39, qui comporte en outre le fait de maîtriser la formation des pores de manière à ce qu'ils présentent une profondeur prédéfinie.

42. Procédé conforme à la revendication 39, qui comporte en outre le fait de maîtriser la formation des pores de manière à ce qu'ils présentent une forme géométrique prédéfinie.

43. Prothèse implantable comprenant :
- une structure de corps de forme globalement cylindrique, présentant une surface externe qui peut entrer en contact avec une surface de lumière interne d'un conduit, ainsi qu'un évidement qui traverse ladite structure de corps dans sa longueur pour y définir une surface interne, cette structure de corps ayant une certaine épaisseur,
- laquelle structure de corps porte de multiples points de dépôt, qui sont formés sur au moins une zone de ladite surface externe et dont la profondeur est inférieure à ladite épaisseur de ladite structure de corps, leur localisation et leur profondeur étant présélectionnées et maîtrisées.

44. Prothèse implantable conforme à la revendication 43, laquelle prothèse est un stent.

45. Prothèse implantable conforme à la revendication 43, dans laquelle lesdits points de dépôt sont des tranchées laser, pratiquées par exposition de ladite surface à une décharge d'énergie provenant d'un laser.

46. Prothèse implantable conforme à la revendication 43, dans laquelle ladite profondeur présélectionnée et maîtrisée vaut de 10 à 90 % de ladite épaisseur.

47. Prothèse implantable conforme à la revendication 43, dans laquelle ladite profondeur présélectionnée et maîtrisée vaut au plus environ 65 % de ladite épaisseur.

48. Prothèse implantable conforme à la revendication 43, dans laquelle lesdits points de dépôt ont une forme présélectionnée.

49. Prothèse implantable conforme à la revendication 48, dans laquelle ladite forme présélectionnée des points de dépôt est globalement cylindrique.

50. Prothèse implantable conforme à la revendication 48, dans laquelle ladite forme présélectionnée des points de dépôt est globalement conique.

51. Prothèse implantable conforme à la revendication 50, dans laquelle lesdits point de dépôt de forme conique présentent une extrémité ouverte et une extrémité fermée, laquelle extrémité ouverte sera au contact de ladite surface de lumière interne de conduit et possède un diamètre inférieur à celui de ladite extrémité fermée.

52. Prothèse implantable conforme à la revendication 43, qui comporte en outre une substance déposée dans lesdits points de dépôt.

53. Prothèse implantable conforme à la revendication 43, qui comporte en outre une substance thérapeutique déposée dans lesdits points de dépôt.

54. Prothèse implantable conforme à la revendication 43, qui comporte en outre une substance thérapeutique déposée dans lesdits points de dépôt, laquelle substance thérapeutique est choisie parmi les antinéoplasiques, antiplaquettaires, anticoagulants, fibrinolytiques, antimitotiques, inhibiteurs de thrombine, anti-inflammatoires et antiproliférants.

55. Prothèse implantable conforme à la revendication 43, qui comporte en outre une substance thérapeutique déposée dans lesdits points de dépôt, laquelle substance thérapeutique est un isotope radioactif.

56. Prothèse implantable conforme à la revendication 43, qui comporte en outre une substance déposée dans lesdits points de dépôt, laquelle substance est un matériau polymère.

57. Prothèse implantable conforme à la revendication 43, qui comporte en outre une substance déposée dans lesdits points de dépôt, laquelle substance est un matériau polymère contenant une substance thérapeutique.

58. Prothèse implantable conforme à la revendication 43, qui comporte en outre une substance déposée dans lesdits points de dépôt, laquelle substance est un matériau polymère contenant une substance thérapeutique, qui est choisie parmi les antinéoplasiques, antiplaquettaires, anticoagulants, fibrinolytiques, antimitotiques, inhibiteurs de thrombine, anti-inflammatoires et antiproliférants.

59. Prothèse implantable conforme à la revendication 43, qui comporte en outre une substance déposée dans lesdits points de dépôt, laquelle substance est un matériau radio-opaque.

60. Prothèse implantable conforme à la revendication 43, qui comporte en outre une première substance thérapeutique déposée dans lesdits points de dépôt, ainsi qu'une couche de matériau polymère formé sur ladite surface dudit corps qui peut entrer en contact avec ladite surface de lumière, laquelle couche de matériau polymère recouvre lesdits points de dépôt et ralentit la libération de ladite première substance thérapeutique hors de ces points de dépôt.

61. Prothèse implantable conforme à la revendication 60, dans laquelle ladite couche de matériau polymère contient une deuxième substance thérapeutique.
